# EUROPEAN PATENT SPECIFICATION

(11) **EP 1 735 029 B1**
(45) Date of publication and mention of the grant of the patent: **30.12.2009**
(21) Application number: 04821990.1
(22) Date of filing: 15.04.2004
(51) Int. Cl.: A61M 1/00

(54) **Method of preparing a surgical handpiece prior to a surgical procedure to reduce air introduction via infusion through the surgial handpiece**
Methode zur Bereitstellung eines chirurgischen Handstücks vor einem chirurgischen Eingriff um das Einführen von Luft durch Infusion durch das Handstück zu reduzieren
Méthode pour la préparation d'une piece à main chirurgicale avant une procedure chirurgicale pour la reduction de l'introduction de l'air via perfusion par la piece à main

(43) Date of publication of application: 27.12.2006
(73) Proprietor: Alcon, Inc., 6331 Hünenberg (CH)
(72) Inventor: Alcon, Inc., 6331 Hünenberg (CH)
(74) Representative: Hanna, Peter William Derek
(86) International application number: PCT/US2004/011673
(87) International publication number: WO 2005/110008

(56) References cited:
- WO-A-96/40026
- WO-A-99/38549
- US-A- 4 369 785
- US-A- 5 242 404
- US-A- 5 380 280
- US-A- 5 580 347
- US-A1- 2001 023 331
- US-A1- 2003 146 299

## Description

### BACKGROUND OF THE INVENTION

### Field of the Invention

The present invention relates to reducing or eliminating the inflow of air into a surgical field that occurs during the time that a surgical instrument is used to introduce fluid into the surgical field.

### Discussion of Related Art

Alcon Laboratories commercializes the SERIES 20000^{®} LEGACY^{®} phacoemulsifier, which is advertised on its website at www.alconlabs.com. The phacoemulsifier includes a console for the physician or medical technician to operate that is programmed to control the operation of a phacoemulsification surgical handpiece by sending appropriate command signals. The applicant is featured on the website with respect to advice that the applicant provides for use with the phacoemulsification surgical handpieces to physicians who use the phacoemulsifier in their medical practice. With respect to infusion, some of the advice found on the website that the applicant provides for the benefit of physicians includes the following:
Although gravity infusion has historically been the standard method for delivering fluid to the eye during phaco, problems with reliabilty and efficiency exist. These are:
   1. Variable infusion rate depending upon fluid volume within the infusion bottle. Fluid cannot leave the infusion bottle unless it is replaced by air, and this is more difficult when the bottle is relatively full. This is so because the air must travel upward through the entire fluid space, which offers resistance to this exchange. In summary, fluid flows from the bottle with somewhat greater difficulty at the beginning of a procedure than it does at the end of the procedure.
   2. The ease with which air enters the infusion bottle is dependent upon the resistance within the "spike vent". This vent has an air filter and in most designs a check valve to prevent liquid leakage. Both of these offer variable resistance to air flow which is in turn manifest as a variation in infusion capacity and resulting perioperative IOP.
Gravity infusion is not the only method by which infusion can be delivered to the eye during phacoemulsification or vitreoretinal prcedures. The GFI^{®} system, currently available with the ALCON^{®} ACCURUS^{®} phacoemulsification and/or vitrectomy system, delivers infusion by maintaining pressure within an infusion source that does not require elevation above the patient's eye level. Infusion flow is delivered more precisely and accurately with such a system, and this permits the surgeon to maintain absolute control over chamber depth with the simple press of a button.
Two brief preparatory steps should be made before insertion of a phaco tip of a phaco-emulsification handpiece.

With the test chamber in place on the tip, hold the handpiece vertically with the tip aimed at the ceiling. Depress the footpedal into position 3 for 2 seconds. This will remove air which may have remained in the handpiece during preparation, and eliminate the sudden shower of air bubbles into the anterior chamber at the beginning of the procedure.

Check to be certain that the infusion line is securely inserted into the handpiece. Then remove the test chamber and depress the footpedal into position 1 to verify that infusion flow is both present and appropriate in degree. The handpiece is now ready for use.

Adequate infusion capacity must be maintained by infusion bottle elevation; a drip chamber level of at least 40 inches (100 cm) above the eye should be present.

After achieving nuclear segmentation (or nucleus flipping for those who prefer this technique), set the flow rate at 40-60 cc/min and 500 mmHg vacuum with the KELMAN Flared MACKOOL ABS tip.

As always, place a second instrument (spatula or chopper) behind the last nucleus segments to be removed in order to protect the posterior capsule in the event of an infusion misdirection syndrome.

U. S. 5,213,569 ('569 patent), recognized the risk of thermal injury to the anterior segment of the eye during the use of phacoemulsification. The '569 patent recognized that the implosion of microbubbles during the process generate massive fluid and shock waves that erode the solid material cataractous nuclei, and can release excess thermal energy into the eye. To prevent heat damage, the '569 patent recommended that a constant flow of balanced salt solution in and out of the anterior segment be provided to transfer heat out of the eye and to remove lens debris (lens milk) so that the surgeon can visualize the area. However, any problem with proper balanced salt solution circulation can quickly result in heat damage to eye tissue. To insure proper circulation, the '569 patent recommended that the surgeon should personally perform, among others, the following two steps:
1. Visually be certain that balanced salt solution is being aspirated from the transparent test chamber into the catchment device, that the test chamber remains filled or only slightly dimpled when the device is in phaco mode and held at eye level, and that balanced salt solution exits from the silicone infusion ports before the device is placed in the anterior chamber;
2. Kink the infusion line while in phaco mode and watch for the test chamber to collapse. Follow this by kinking the aspiration line and listen for the sound of vacuum build up.

Reference is also made to US- 5,580,347 (Reimels H.G.), US-2003/146,299-A (Suzuki Nobuo et al), WO 99/38549-A (Mentor Corp.), US-4,369,785 (Rehkopf P.G. et al) as being representative of the prior art.

The present applicant observes that the introduction of air bubbles into the eye is very common during the ultrasonic procedure known as phacoemulsification, and also during non-ultrasonic removal of lens material by irrigation and aspiration. The applicant has performed tens of thousands of phacoemulsification and irrigation/aspiration procedures, and has been able to identify the source of the problem which is as follows.

After preparation of the handpiece for insertion into the eye, air enters the distal portion of the handpiece from the atmosphere either 1) immediately upon the removal of the "test chamber" (a malleable chamber that surrounds the tip of the handpiece and isolates it from the atmosphere), or 2) as the tip is lowered toward the eye and fluid passively drains from the infusion channels in the distal portion of the handpiece and is replaced by atmospheric air. The air travels to the highest portion of the infusion channel to which it can gain access, and remains there until such time during the surgical procedure when it is carried into the eye by infusion fluid. This may occur early in the procedure, but it can be delayed if relatively low infusion flow does not create enough force on the air bubble to pull it away from its tenuous attraction to the inner wall of the infusion channel. It would be desirable to prevent air from entering into the handpiece in the first place, and thereby eliminate the problem.

Although the problem could be eliminated by the maintenance of constant flow of infusion after preparation of the handpiece and beginning immediately with removal of the above described test chamber, this creates the problem of wide dispersion of fluid onto the surgical field as the handpiece approaches the eye. Such dispersion occurs because the usual pressure head within the infusion system (40 - 90 mm Hg) drives infusion fluid forcefully from the handpiece. This often causes the surgeon to have poor visibility of the surgical organ, or causes the patient to be startled by the sudden rush of fluid onto the portion of the body to be operated upon. Furthermore, the ultrasonic instrument is prepared with a test chamber in place, and even if infusion is activated prior to removal of the test chamber, there is a sudden escape of the pressurized fluid into the atmosphere when the test chamber is removed, and air is therefore still able to gain access into the distal infusion channel as a replacement to the exit of the pressurized infusion fluid.

### SUMMARY OF THE INVENTION

The present invention provides a method of preparing a surgical handpiece prior to a surgical procedure to reduce air introduction via infusion through a surgical handpiece, in accordance with claims which follow. The introduction of air into an infusion line may be reduced or eliminated by lowering the pressure of an infusion fluid.

Thereafter, the infusion line may be partially occluded or fully opened and then the test chamber may be removed to permit a constant, low flow rate of infusion through a surgical handpiece.

### BRIEF DESCRIPTION OF THE DRAWING

For a better understanding of the present invention, reference is made to the following description and accompanying drawings, while the scope of the invention is set forth in the appended claims:
Fig. 1 is a schematic representation of a sequence of steps part of which exemplify the invention.
Fig. 2 is a schematic representation of the equipment used for carrying out the sequence of Fig. 1.

### DETAILED DESCRIPTION OF THE DRAWING

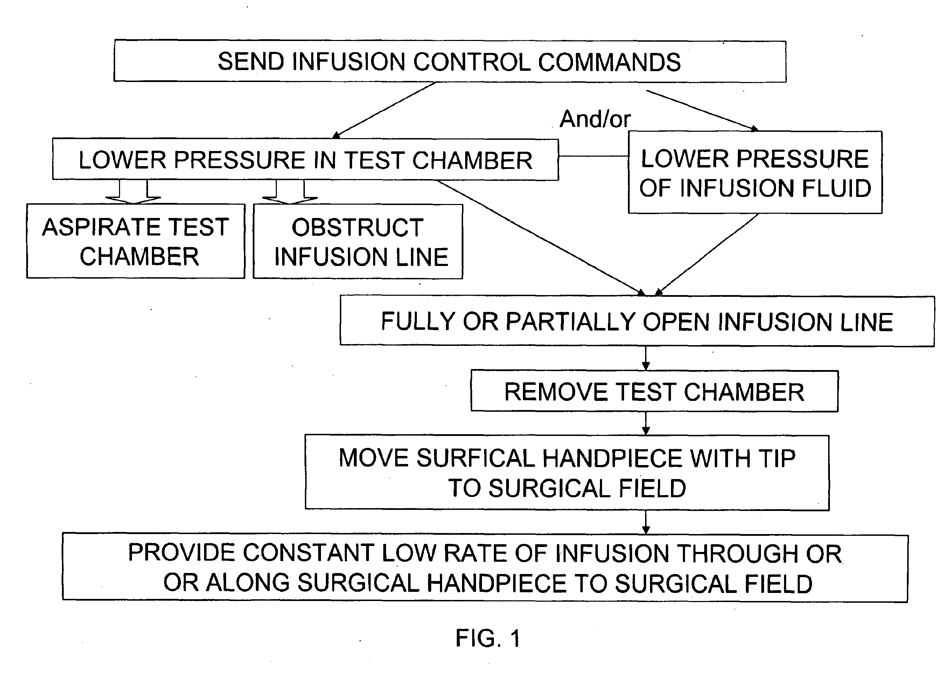

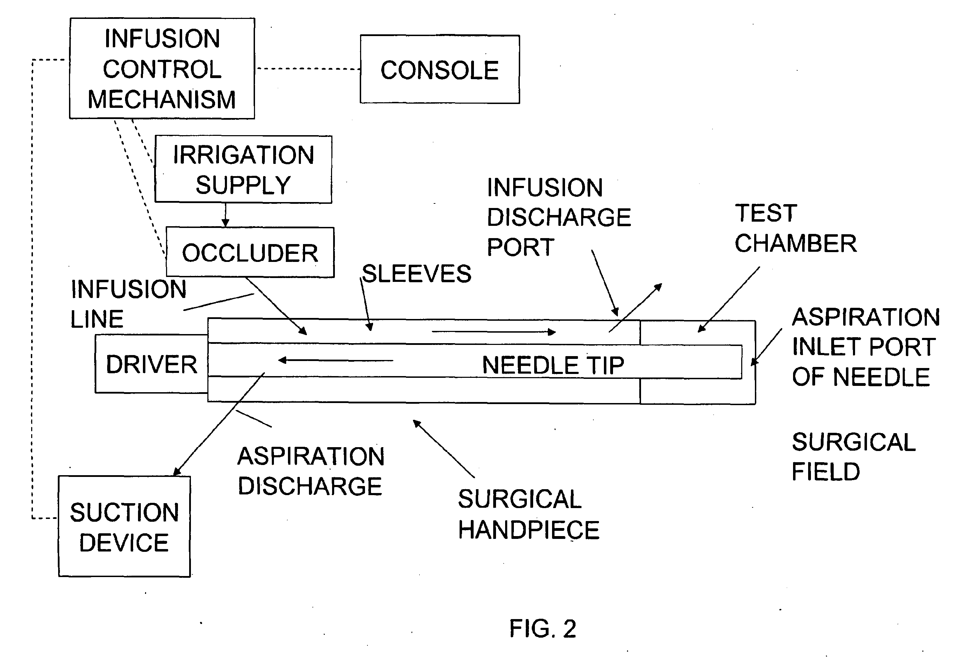

Turning to the drawing, Fig. 1 illustrates the recommended sequence of steps and Fig. 2 illustrates the equipment used.

To eliminate the problem of the introduction of air bubbles, the console can be activated to "prepare the handpiece for insertion" by automatically sending a signal to the infusion control mechanism to issue commands to appropriate drivers to reduce the infusion pressure. The drivers may be part of an irrigation supply or an occluder to open or close the infusion line and partially or substantially occlude the infusion line.

The infusion line may then be partially or fully opened prior to removal of the test chamber to provide a constant low rate of infusion through the surgical handpiece.

The infusion pressure may be reduced by the action of an appropriate driver to reduce infusion pressure, such as by either reducing the height of the infusion fluid supply (gravitational infusion supply) or decreasing the pressure within the infusion supply (pressurized infusion supply). Preferably, the infusion pressure is lowered to a level less than the 40 - 90 mmHg pressure head within the infusion line.

The result of this action is to create a constant low rate of infusion when the test chamber is subsequently removed. This constant dripping of infusion fluid does not permit air to enter the infusion line, e. g., the distal infusion pathway.

The handpiece (including the tip) are then moved into the surgical field and the tip is introduced into the organ. Immediately thereafter, the surgeon may activate the console to a previously programmed state whereby the partial occlusion line obstruction is eliminated, a greater infusion pressure head is present, and footpedal depression by the surgeon would result in activation of a pump of a suction device, with tissue removal, tip vibration or oscillation, etc.

The infusion line or channel may irrigate the surgical field with a balanced salt solution, saline solution or the like. The needle tip is vibrated or oscillated by a driver. The tissue removal arises by sucking the tissue through the aspiration inlet port through the tip and needle to pass through the aspiration discharge to the suction device.

The needle may or may not have one or more exterior sleeves that extend along its length up to the proximal portion of the tip. Irrigation fluid may flow along the exterior surface of the needle or, if two concentrically arranged sleeves are provided, through passage between the sleeves.

While the foregoing description and drawings represent the preferred embodiments of the present invention, it will be understood that various changes and modifications may be made without departing from the scope of the present invention as defined in the appended claims.

## Claims

1. A method of preparing a surgical handpiece prior to a surgical procedure to reduce air introduction via infusion through a surgical handpiece, comprising;
applying a test chamber to enclose a needle tip of the handpiece to isolate it from the atmosphere,
generating commands;
in response to the commands, causing a lowering of infusion fluid pressure;
**characterized by**,
removing the test chamber from the needle tip; and
as a consequence of causing the lowering of the infusion fluid pressure and upon removal of the test chamber from the needle tip, creating a constant low rate of flow of an infusion fluid to the needle tip.

2. A method as in claim 1, wherein reducing pressure of the infusion fluid includes reducing an elevation level of an infusion supply to an infusion line.

3. A method as in claim 1, wherein reducing pressure of the infusion fluid includes decreasing a pressure within an infusion supply to an infusion line that extends along the surgical handpiece.

4. A method as in claim 1, wherein the causing the lowering of the infusion pressure includes partially opening an infusion line by partially removing an obstruction in the infusion line so that the obstruction at most substantially, yet not fully, occludes the infusion line.

5. A method as in claim 4, wherein causing the lowering of the infusion pressure includes reducing an elevation level of an infusion supply to the infusion line.

6. A method as in claim 4, wherein causing the lowering of the infusion pressure includes decreasing a pressure within an infusion supply to the infusion line.

7. A method as in claim 4, further comprising removing the obstruction from the infusion line to increase an infusion pressure head, and irrigating through the infusion line with the increased infusion pressure head, and vibrating or oscillating the needle of the surgical handpiece while carrying out the irrigating.

## Patentansprüche

1. Verfahren zum Vorbereiten eines chirurgischen Handstücks vor einem chirurgischen Eingriff, um die Einleitung von Luft über eine Infusion durch ein chirurgisches Handstück zu verringern, mit:
Anbringen einer Prüfkammer, so dass die Nadelspitze des Handstücks umschlossen ist, um sie gegenüber der Atmosphäre zu isolieren, Erzeugen von Befehlen;
als Antwort auf die Befehle, Bewirken einer Verringerung des Drucks eines Infusionsfluids;
**gekennzeichnet durch**
Entfernen der Prüfkammer von der Nadelspitze; und
als Folge der Bewirkung der Druckverringerung des Infusionsfluids und bei Entfernen der Prüfkammer von der Nadelspitze Erzeugen eines konstanten niedrigen Durchsatzes des Infusionsfluids zur Nadelspitze.

2. Verfahren nach Anspruch 1, bei dem die Druckverringerung des Infusionsfluids die Absenkung des Höhenniveaus einer Infusionsversorgung zur Infusionsleitung enthält.

3. Verfahren nach Anspruch 1, bei dem die Druckverringerung des Infusionsfluids die Druckverringerung in der Infusionsversorgung zur Infusionsleitung enthält, die entlang dem chirurgischen Handstück verläuft.

4. Verfahren nach Anspruch 1, bei dem die Verringerung des Infusionsdrucks die teilweise Öffnung einer Infusionsleitung enthält, indem ein Hindernis in der Infusionsleitung teilweise entfernt wird, so dass das Hindernis höchstens im Wesentlichen, aber nicht vollständig, die Infusionsleitung sperrt.

5. Verfahren nach Anspruch 4, bei dem die Verringerung des Infusionsdrucks die Absenkung des Höhenniveaus einer Infusionsversorgung zur Infusionsleitung enthält.

6. Verfahren nach Anspruch 4, bei dem die Verringerung des Infusionsdrucks die Druckverringerung in einer Infusionsversorgung zur Infusionsleitung enthält.

7. Verfahren nach Anspruch 4, das ferner die Entfernung des Hindernisses aus der Infusionsleitung, um die Infusionsdruckhöhe zu vergrößern, und die Spülung durch die Infusionsleitung bei der vergrößerten Infusionsdruckhöhe sowie die Schwingung oder Oszillation der Nadel des chirurgischen Handstücks aufweist, während die Spülung ausgeführt wird.

## Revendications

1. Procédé de préparation d'une pièce à main chirurgicale avant une procédure chirurgicale afin de réduire l'introduction d'air via une perfusion à travers la pièce à main chirurgicale, comprenant les étapes consistant à :
mettre en place une chambre d'analyse en entourant la pointe d'aiguille de la pièce à main afin de l'isoler de l'atmosphère,
générer des commandes ;
en réponse aux commandes, provoquer une diminution de la pression de liquide de perfusion ;
**caractérisé par**
un retrait de la chambre d'analyse depuis la pointe d'aiguille ; et
en conséquence de la diminution de la pression de liquide de perfusion et après retrait de la chambre d'analyse depuis la pointe d'aiguille, une création d'un faible débit constant d'un liquide de perfusion à la pointe d'aiguille.

2. Procédé selon la revendication 1, dans lequel la réduction de la pression du liquide de perfusion comprend la réduction d'un niveau d'élévation d'une alimentation de perfusion à un tube de perfusion.

3. Procédé selon la revendication 1, dans lequel la réduction de la pression du liquide de perfusion comprend la diminution d'une pression dans une alimentation de perfusion vers un tube de perfusion qui s'étend le long de la pièce à main chirurgicale.

4. Procédé selon la revendication 1, dans lequel l'action de provoquer la diminution de la pression de perfusion comprend une ouverture partielle du tube de perfusion par le retrait partiel d'une obstruction dans le tube de perfusion afin que l'obstruction occlue au plus essentiellement, mais pas complètement, le tube de perfusion.

5. Procédé selon la revendication 4, dans lequel l'action de provoquer la diminution de la pression de perfusion comprend la réduction d'un niveau d'élévation d'une alimentation de perfusion vers le tube de perfusion.

6. Procédé selon la revendication 4, dans lequel l'action de provoquer la diminution de la pression de perfusion comprend la baisse d'une pression dans une alimentation de perfusion vers le tube de perfusion.

7. Procédé selon la revendication 4, comprenant en outre un retrait de l'obstruction depuis le tube de perfusion afin une hauteur manométrique de pression de perfusion, et une irrigation à travers le tube de perfusion avec la hauteur manométrique de perfusion augmentée, et une vibration ou une oscillation de l'aiguille de la pièce à main chirurgicale pendant la mise en oeuvre de l'irrigation.
